# EUROPÄISCHE PATENTANMELDUNG

(11) **EP 0 542 037 A1**
(43) Veröffentlichungstag der Anmeldung: **19.05.1993**
(21) Anmeldenummer: 92118399.2
(22) Anmeldetag: 28.10.1992
(51) Int. Cl.: C07D 307/88

(54) **Verfahren zur Herstellung von Phthaliden**

(30) Priorität: 11.11.1991 DE 4136992
(71) Anmelder: BASF Aktiengesellschaft, D-67063 Ludwigshafen (DE)
(72) Erfinder: Henkelmann, Jochem, Dr., W-6700 Ludwigshafen (DE); Ruehl, Thomas, Dr., W-6700 Ludwigshafen (DE); Zimmermann, Horst, Dr., W-6800 Mannheim 24 (DE)

(57) **Zusammenfassung**

Verfahren zur Herstellung von Phthaliden der allgemeinen Formel I 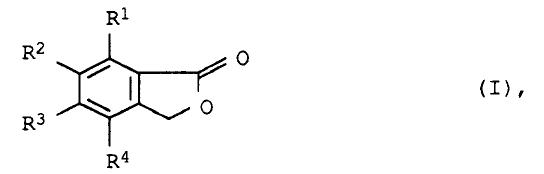
in der R¹, R², R³ und R⁴ unabhängig voneinander Wasserstoff, C₁- bis C₄-Alkyl und C₁- bis C₄-Alkoxy bedeuten, durch Umsetzung von Phthalsäureanhydride der allgemeinen Formel II 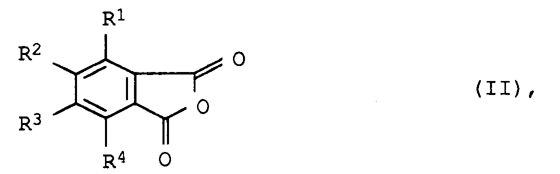
in der die Reste R¹, R², R³ und R⁴ die oben genannten Bedeutungen haben, an Hydrierkatalysatoren bei Temperaturen von 50 bis 400°C und Drücken von 1 bis 400 bar, indem man Hydrierkatalysatoren im Wesentlichen in Abwesenheit von Lewis-Säuren einsetzt.

## Beschreibung

Die vorliegende Erfindung betrifft ein Verfahren zur Herstellung von Phthaliden durch katalytische Hydrierung von Phthalsäureanhydriden an Festbettkatalysatoren, die im Wesentlichen frei von Lewis-Säuren sind.

Aus der US-A-4,485,246 und der US-A-3,957,825 ist bekannt, daß man Phthalid aus Phthalsäureanhydrid durch Hydrierung mit homogenen Rutheniumkatalysatoren herstellen kann.

Aus der DE-A-32 01 300 ist die katalytische Hydrierung von Phthalsäureanhydrid mit Nickel als Katalysator und Benzoesäuremethylester als Lösungsmittel bekannt.

Aus der US-A-4,973,713 ist ein Verfahren bekannt, das von Phthalsäureanhydrid ausgeht, das - unter Zusatz von Eisenchlorid - diskontinuierlich an Palladium/Rheniumkatalysatoren hydriert wird.

Die bislang verwendeten Hydrierkatalysatoren wurden als Suspensions- bzw. als Homogenkatalysatoren eingesetzt, was bei der Aufarbeitung zu Schwierigkeiten bei deren Abtrennung führt.

Der vorliegenden Erfindung lag daher die Aufgabe zugrunde, den zuvor genannten Nachteilen abzuhelfen.

Demgemäß wurde ein neues und verbessertes Verfahren zur Herstellung von Phthaliden der allgemeinen Formel I 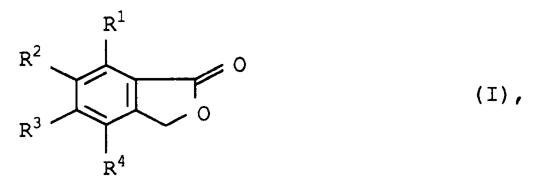
in der R¹, R², R³ und R⁴ unabhängig voneinander Wasserstoff, C₁- bis C₄-Alkyl und C₁- bis C₄-Alkoxy bedeuten, durch Umsetzung von Phthalsäureanhydride der allgemeinen Formel II 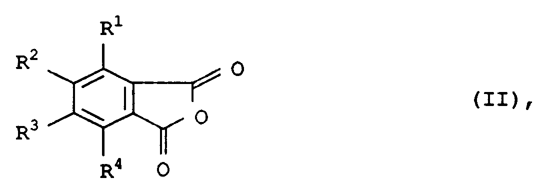
in der die Reste R¹, R², R³ und R⁴ die oben genannten Bedeutungen haben, an Hydrierkatalysatoren bei Temperaturen von 50 bis 400°C und Drücken von 1 bis 400 bar gefunden, welches dadurch gekennzeichnet ist, daß man als Hydrierkatalysatoren Festbettkatalysatoren im Wesentlichen in Abwesenheit von Lewis-Säuren einsetzt.

Die erfindungsgemäße katalytische Hydrierung läßt sich wie folgt durchführen:
Man kann das Phthalsäureanhydrid II, den Hydrierkatalysator und gegebenenfalls ein inertes Lösungsmittel in einem Druckbehälter vorlegen, das Ausgangsgemisch aufheizen und Wasserstoff gegebenenfalls aufpressen.

Ferner kann man den Hydrierkatalysator in einem Rohrreaktor als Festbett vorlegen und das Phthalsäureanhydrid II, gegebenenfalls gelöst in einem inerten Lösungsmittel, mit dem Wasserstoff in Sumpf- oder Rieselfahrweise durch den Reaktor führen.

Neben den Anhydriden selbst können auch die entsprechende Dicarbonsäuren, Mono- und Diester oder Gemische eingesetzt werden. Alle diese Einsatzstoffe können in fester, flüssiger oder gasförmiger Form in das erfindungsgemäße Verfahren eingebracht werden. Besonders bevorzugt ist die Verwendung von Phthalsäureanhydrid.

Die Hydrierung kann bei Temperaturen von 50 bis 400°C, bevorzugt 150 bis 250°C und Drücken von 1 bis 400 bar, bevorzugt 50 bis 400 bar, besonders bevorzugt 100 bis 300 bar durchgeführt werden. Die Reaktionstemperatur hängt jedoch stark von der Art des verwendeten Katalysators ab.

Man kann diskontinuierlich, insbesondere aber kontinuierlich, in der Sumpf- oder Rieselfahrweise arbeiten, wobei der Katalysator fest, z.B. als Festbettkatalysator angeordnet ist. Dabei haben sich Katalysator belastungen von 0,01 bis 1, insbesondere 0,05 bis 0,4 kg Phthalsäureanhydrid II je Liter Katalysator und Stunde bewährt.

Als Reaktoren können z.B. Rohrreaktoren oder Rohrbündelreaktoren verwendet werden.

Als Hydrierkatalysatoren eignen sich bevorzugt Oxide der ersten bis vierten Hauptgruppe des Periodensystems der Elemente wie Lithium, Natrium, Kalium, Calcium, Bor, Aluminium, Silicium und Zinn, bevorzugt Zinn, Bor und Aluminium, bevorzugt Zinn und Bor ausgenommen Sauerstoff, der ersten bis achten Nebengruppe des Periodensystems der Elemente wie Titan, Zirkon, Vanadin, Niob, Chrom, Molybdän, Mangan, Rhenium, Cobalt, Nickel, Ruthenium, Rhodium, Palladium, Osmium, Iridium, Platin, Kupfer und Silber, bevorzugt Cobalt, Ruthenium, Palladium, Kupfer und Silber, besonders bevorzugt Palladium, Kupfer und Silber und der Lanthanidengruppe wie Lanthan, Praseodym, Samarium und Ytterbium, bevorzugt Praseodym oder deren Gemische.

Ferner eignen sich metallische Kontakte auf Trägermaterialien. Als Metalle eignen sich Ruthenium, Rhenium, Palladium, Platin, Bor und Zinn, bevorzugt Palladium, Rhenium, Ruthenium, Zinn und Bor, besonders bevorzugt Palladium und Rhenium.

Die Hydrierkatalysatoren enthalten beispielsweise 0 bis 70 Gew.-% CuO, bevorzugt 15 bis 60 Gew.-% CuO, 0 bis 99 Gew.-% CoO, bevorzugt 50 bis 99 Gew.-% CoO, 0 bis 70 Gew.-% NiO, bevorzugt 20 bis 60 Gew.-% NiO, 0 bis 50 Gew.-% ZrO₂, bevorzugt 5 bis 30 Gew.-% ZrO₂, 0 bis 70 Gew.-% Cr₂O₃, bevorzugt 30 bis 70 Gew.-% Cr₂O₃, 0 bis 99 Gew.-% Al₂O₃, bevorzugt 40 bis 99 Gew.-% Al₂O₃, 0 bis 99 Gew.-% SiO₂, bevorzugt 50 bis 90 Gew.-% SiO₂, 0 bis 99 Gew.-% Kohle - z.B. als Aktivkohle -, bevorzugt 80 bis 99 Gew.-% Kohle, 0 bis 20 Gew.-% MoO, bevorzugt 1 bis 15 Gew.-% MoO, 0 bis 20 Gew.-% MnO₂, bevorzugt 1 bis 10 Gew.-% MnO₂, 0 bis 10 Gew.-% PrO₂, bevorzugt 1 bis 7 Gew.-% PrO₂, 0 bis 20 Gew.-% AgO, bevorzugt 1 bis 15 Gew.-% AgO, 0 bis 30 Gew.-% Na₂O, bevorzugt 0,1 bis 20 Gew.-% Na₂O, 0 bis 10 Gew.-% PdO, bevorzugt 0,1 bis 10 Gew.-% PdO, 0 bis 10 Gew.-% Ru, bevorzugt 0,1 bis 5 Gew.-% Ru, 0 bis 10 Gew.-% Re, bevorzugt 0,1 bis 10 Gew.-% Re, 0 bis 10 Gew.-% Pd, bevorzugt 0,1 bis 10 Gew.-% Pd, 0 bis 10 Gew.-% Sn, bevorzugt 0,1 bis 5 Gew.-% Sn und 0 bis 10 Gew.-% B, bevorzugt 0,1 bis 5 Gew.-% B.

Die Katalysatoren können sowohl als Trägerkatalysator oder in kompakter Form, d.h. ohne Träger eingesetzt werden. Die Gewichtsprozentzahlen addieren sich mit den Trägermaterialen zu 100 Gew.-%.

Als Trägermaterialien können übliche Materialien verwendet werden. So eignen sich beispielsweise Bims, Silciumdioxid, Aluminiumoxid, Titandioxid, Aktivkohle, Silikate und Zeolithe bevorzugt Aktivkohle, Aluminiumoxid, Siliciumdioxid und Titandioxid, besonders bevorzugt Aktivkohle, Aluminiumoxid und Siliciumdioxid.

Gegebenenfalls können zur Herstellung der Katalysatoren Bindemittel oder Formhilfsmittel wie Natriumcarbonat, Kaliumcarbonat, Natriumoxid und Magnesiumoxid, bevorzugt Natriumoxid mitverwendet werden.

Als inerte Lösungsmittel eignen sich z.B. Ether wie Diethylether und Tetrahydrofuran, bevorzugt Tetrahydrofuran, Ester wie Essigsäureethylester und Benzoesäuremethylester, Lactone wie Butyrolacton, bevorzugt Butyrolacton, Alkohole wie Ethanol, Butanol und Propanol, bevorzugt Butanol und Wasser. Bevorzugte Lösungsmittelgruppen sind Ether, Ester und Lactone.

Die Aufarbeitung erfolgt nach üblichen Methoden wie Destillation.

Die Substituenten R¹, R², R³ und R⁴ in den Verbindungen I und II haben folgende Bedeutungen:
- unabhängig voneinander
- Wasserstoff,
- C₁- bis C₄-Alkyl wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
- C₁- bis C₄-Alkoxy wie Methoxy, Ethoxy, n-Propoxy, iso-Propoxy, n-Butoxy, iso-Butoxy, sec.-Butoxy und tert.-Butoxy, bevorzugt Methoxy und Ethoxy, besonders bevorzugt Methoxy,
besonders bevorzugt bedeuten alle Substituenten R¹, R², R³ und R⁴ Wasserstoff.

Die Phthalsäureanhydride II sind allgemein bekannt, wie das Phthalsäureanhydrid, oder können nach bekannten Verfahren aus Benzoesäurederivaten, erhalten werden (JOC; 51, 3439 bis 3446 (1986), Synthesis; 223 bis 224 (1985)).

Die Phthalide I eignen sich z.B. als Ausgangsstoff für die Synthese von Wirkstoffen für den Pflanzenschutz.

### Beispiele

Die in den Beispielen verwendeten Katalysatoren waren wie folgt zusammengesetzt (Angaben in Gew.-%):

| Katalysator | Zusammensetzung |
|---|---|
| A | 98 % CoO; 0,1 % Na₂O |
| B | 25 % NiO; 5 % ZrO₂; 70 % SiO₂ |
| C | 50 % NiO; 50 % Cr₂O₃ |
| D | 0,5 % PdO; 94,5 % Al₂O₃; 5 % PrO₂ |
| E | 5 % AgO; 0,4 % PdO; 1,4 % MnO₂; 93 % Al₂O₃ |
| F | 1 % Ru; 1,2 % Sn; 1,3 % B; 96,5 % Al₂O₃ |
| G | 52 % CuO; 48 % Al₂O₃ |
| H | 3 % Pd; 3 % Re; 94 % Kohle |
| I | 64 % CoO; 18 % CuO; 7 % MnO₂; 4 % MoO; 9,2 % Na₂O |
| K | 50 % NiO; 30 % ZrO₂, 18 % CuO; 1,5 % MoO₂; 0,2 % Na₂O |

### Beispiel 1

Die hydrierende Umsetzung wurde in einem 300-ml-Rührautoklav, in dem sich 5 g des Kontaktes A, 50 g Phthalsäureanhydrid und 50 ml Tetrahyd rofuran befanden, durchgeführt. Bei einem Gesamtdruck von 260 bar undeiner Temperatur von 200°C wurde 12 h hydriert.

Die gaschromatographische Untersuchung des flüssigen Reaktionsaustrages ergab eine Ausbeute an Phthalid von 84 %. Weiterhin wurden 7,5 % Phthalsäureanhydrid gefunden.

### Beispiel 2 bis 10

Die Hydrierung wurde analog Beispiel 1 durchgeführt. Die jeweils verwendeten Katalysatoren und Reaktionsbedingungen sind zusammen mitder Zusammensetzung an Phthalsäureanhydrid und Phthalid in Tabelle 1 wiedergegeben.

**Tabelle 1**

| Katalysator | Temperatur [°C] | Phthalsäureanhydrid [GC-Fl.-%] | Phthalid [GC-Fl.-%] |
|---|---|---|---|
| B | 250 | - | 71 |
| C | 250 | 15 | 72 |
| D | 250 | - | 86 |
| E | 250 | - | 89 |
| F | 250 | 5,5 | 80 |
| G | 200 | 44 | 51 |
| H | 200 | 27 | 59 |
| I | 200 | 37 | 14 |
| K | 150 | 8,3 | 58 |

### Beispiel 11

Die hydrierende Umsetzung wurde in einem Rohrreaktor (Länge 2000 mm, Durchmesser, 16 mm), in dem sich der Kontakt D in einem Festbett befand, durchgeführt. Der Reaktor wurde über einen außenliegenden Heizmantel mit Öl auf die Reaktionstemperatur aufgeheizt. Die gasförmigen und flüssigen Einsatzstoffe durchströmten den Reaktor von obe nach unten (Rieselfahrweise). Der Hydrieraustrag wurde entspannt und in einem Gas-Flüssigkeits-Abscheider in seine gasförmigen und flüssigen Bestandteile zerlegt.

Der Katalysator wurde in Form von 2 bis 4 mm großem Splitt eingesetzt und vor Beginn der Hydrierung mit Wasserstoff aktiviert.

Bei einem Gesamtdruck von 300 bar und einer Temperatur von 230°C wurden dem Reaktor stündlich 0,25 kg Phthalsäureanhydrid je Liter Katalysator in Form einer 10 %igen Lösung in Tetrahydrofuran sowie 0,5 m³ Wasserstoff je kg Katalysator zugeführt.

Die gaschromatographische Untersuchung des flüssigen Reaktionsaustrages ergab einen Umsatz von 84 % bei einer Selektivität von 88 %.

### Beispiel 12 bis 13

Die hydrierenden Umsetzungen wurden analog Beispiel 11 durchgeführt. Die jeweils verwendeten Katalysatoren und Reaktionsbedingungen sind zusammen mit dem Umsatz und Selektivität in Tabelle 2 wiedergegeben.

**Tabelle 2**

| Katalysator | Katalysatorbelastung [kg/l x h] | Temp. [°C] | Umsatz [GC-Fl.-%] | Selektivität [GC-Fl.-%] |
|---|---|---|---|---|
| E | 0,12 | 240 | 96 | 89 |
| I | 0,06 | 170 | 86 | 64 |

### Beispiel 14 bis 17

Die hydrierende Umsetzung wurde analog Beispiel 11 durchgeführt, nur daß die Hydrierungen in einem Reaktor mit 2000 mm Länge und 45 mm Durchmesser durchgeführt wurden.

Die jeweils verwendeten Katalysatoren und Reaktionsbedingungen sind zusammen mit dem Umsatz und Selektivität in Tabelle 3 wiedergegeben.

**Tabelle 3**

| Katalysator | Katalysatorbelastung [kg/l x h] | Temp. [°C] | Umsatz [GC-Fl.-%] | Selektivität |
|---|---|---|---|---|
| H | 0,06 | 180 | 93 | 95 |
| F | 0,12 | 240 | 83 | 52 |
| G | 0,25 | 210 | 68 | 96 |
| K | 0,04 | 100 | 72 | 72 |

## Patentansprüche

1. Verfahren zur Herstellung von Phthaliden der allgemeinen Formel I 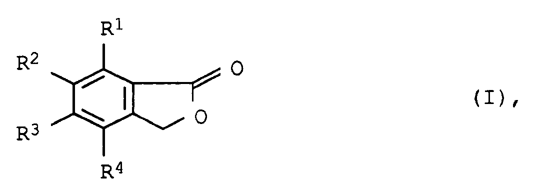 in der R¹, R², R³ und R⁴ unabhängig voneinander Wasserstoff, C₁- bis C₄-Alkyl und C₁- bis C₄-Alkoxy bedeuten, durch Umsetzung von Phthalsäureanhydride der allgemeinen Formel II 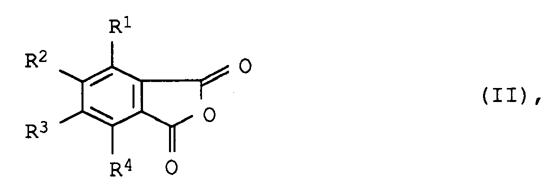 in der die Reste R¹, R², R³ und R⁴ die oben genannten Bedeutungen haben, an Hydrierkatalysatoren bei Temperaturen von 50 bis 400°C und Drücken von 1 bis 400 bar, dadurch gekennzeichnet, daß man Hydrierkatalysatoren im Wesentlichen in Abwesenheit von Lewis-Säuren einsetzt.

2. Verfahren zur Herstellung von Phthaliden der allgemeinen Formel I nach Anspruch 1, dadurch gekennzeichnet, daß man die Umsetzung bei Temperaturen von 150 bis 250°C durchführt.

3. Verfahren zur Herstellung von Phthaliden der allgemeinen Formel I nach Anspruch 1, dadurch gekennzeichnet, daß man die Umsetzung bei Drücken von 50 bis 400 bar durchführt.

4. Verfahren zur Herstellung von Phthaliden der allgemeinen Formel I nach Anspruch 1, dadurch gekennzeichnet, daß man als Hydrierkatalysatoren Oxide der ersten bis vierten Hauptgruppe des Periodensystems der Elemente, ausgenommen Sauerstoff, der ersten bis achten Nebengruppe des Periodensystems der Elemente und der Lanthanidengruppe oder deren Gemische sowie metallische Kontakte auf Trägermaterialien einsetzt.
